# EUROPEAN PATENT SPECIFICATION

(11) **EP 3 539 618 B1**
(45) Date of publication and mention of the grant of the patent: **25.08.2021**
(21) Application number: 19161049.2
(22) Date of filing: 06.03.2019
(51) Int. Cl.: A61Q 1/02, A61K 8/81, A61K 8/891, A61K 8/895, A61K 8/02, A61K 8/04, A61K 8/14

(54) **A COSMETIC PRODUCT AND A METHOD OF PRODUCING SAID COSMETIC PRODUCT**
KOSMETIKPRODUKT UND VERFAHREN ZUR HERSTELLUNG DES BESAGTEN KOSMETIKPRODUKTS
PRODUIT COSMÉTIQUE ET PROCÉDÉ DE PRODUCTION DUDIT PRODUIT COSMÉTIQUE

(30) Priority: 14.03.2018 IT 201800003541
(43) Date of publication of application: 18.09.2019
(73) Proprietor: Chromavis S.p.A., 20122 Milano (IT)
(72) Inventor: Morosini, Ambra, 26013 Crema (IT); Menna, Cristina, 20100 Milano (IT)
(74) Representative: Vanosi, Adelio Valeriano

(56) References cited:
- KR-A- 20120 085 361
- KR-A- 20150 078 973
- US-A1- 2015 272 861
- Anonymous: "BRB Caprylyl Methicone", , 1 January 2016 (2016-01-01), XP055504158, Retrieved from the Internet: URL:http://dewolfchem.com/wp-content/uploa ds/2016/03/BRB-Caprylyl-Methicone.pdf [retrieved on 2018-09-04]

## Description

### FIELD OF THE INVENTION

The present invention relates to a cosmetic product and to a method for producing the same.

In particular, the invention relates to a cosmetic product for make-up, for example a product for lips, eyes, face, body, such as a foundation, eye shadow, blusher, highlighting powder, bronzer, or a lip balm.

### BACKGROUND ART

Some makeup products, such as lipsticks or lip balms, are marketed in the form of elongated sticks housed in conventional similar machines, or more recently, in the form of a small cylinder with a diameter and height of 2-4 cm with a hemispherical surface conformation, secured to a container equipped with a fastening.

In order to attract consumers, various containers have been designed over time, some of which feature very original conformations and therefore prove to be attractive in themselves, regardless of the make-up product contained therein.

There are also commonly known make-up products for the same purpose, mostly consisting of very dense powders or pastes, which are offered in the form of solid monochromatic or multicoloured spheres, to be used for example as face powders or bronzers. US 2015/272861 discloses dispersion of droplets having size preferably less than 0.5 mm of a mixture of dimethicone and dimethicone crosspolymer suspended in an aqueous gel thickened by the carbomer polymer.

As fashions change, consumers - who are increasingly demanding - want innovative makeup products that provide original or specific makeup effects, but at the same time are also extremely attractive from an aesthetical point of view, with the aim to encourage consumers to choose and purchase them.

As a result, it is felt the need for make-up products that are aesthetically attractive in themselves, while offering high cosmetic qualities, stability over time, and skin tolerability.

### SUMMARY OF THE INVENTION

The object of the present invention is to produce a cosmetic product, in particular a makeup product, featuring an attractive, original appearance, which can be appreciated from both an aesthetic and a functional point of view upon application thereof.

This and other objects are achieved by producing a cosmetic product according to the technical teachings of the claims annexed hereto.

One advantage of the cosmetic product according to the invention is that said product offers attractive aesthetic properties which remain stable over time, guaranteeing equally high cosmetic qualities and skin tolerability.

### BRIEF DESCRIPTION OF THE FIGURES

Further characteristics and advantages of the invention will become apparent in the description of a preferred but not exclusive embodiment of the invention, illustrated by way of a non-limiting example in the drawings annexed hereto, wherein:
Figure 1 shows a side view of a cosmetic produced according to Example 1, in a transparent glass container with a dispensing cap; and
Figure 2 is an enlargement of the portion enclosed within the box shown with a dashed line in Figure 1.

### DETAILED DESCRIPTION OF THE INVENTION

The cosmetic product according to the present invention comprises spheres of an anhydrous composition suspended in an aqueous gel, wherein:
said anhydrous composition comprises 10-65 wt% of a mixture of dimethicone and dimethicone crosspolymer, based on the weight of the anhydrous composition, and optionally at least one pigment, and
said aqueous gel comprises water and polyacrylate, said aqueous gel having a Brookfield viscosity of 0.2-0.9 Pa*s, at 20 rpm and 25°C.

The term "sphere" or "spheres" refers not only to perfectly spherical solid forms but also solid forms that are essentially spherical or are comparable to spheres, such as spheroids.

Preferably, the spheres of anhydrous composition are present in the cosmetic product in an amount by weight which is less than or equal to the aqueous gel. More preferably, the anhydrous composition spheres are present in the cosmetic product in amounts no greater than 50 wt%, based on the weight of the cosmetic product.

Preferably, in the mixture of dimethicone and dimethicone crosslinked polymer (also known in the industry as "dimethicone crosspolymer"), said dimethicone is a low-viscosity dimethicone, more preferably with a viscosity of 5-1 CTS.

Preferably, in said mixture, dimethicone is in an amount greater than dimethicone crosslinked polymer.

In preferred embodiments, in said mixture, dimethicone and dimethicone crosslinked polymer are in a weight ratio of 20:1 to 2:1, more preferably 10:1 to 2:1, and even more preferably 8:1 to 3:1.

Preferably, the mixture of dimethicone and dimethicone crosslinked polymer has a kinematic viscosity of 300-600 m²/s, at 25 °C.

Preferably, said anhydrous composition comprises 10-60 wt% of a mixture comprising dimethicone and dimethicone crosslinked polymer, based on the weight of the anhydrous composition. The percentage of the mixture in the anhydrous composition depends on the intended use of the cosmetic product. Indeed, the higher the percentage of the mixture in the anhydrous composition, the lower the amount of pigment released, when present. Therefore, by modulating the percentage of the mixture in the anhydrous composition, different chromatic effects can be obtained.

Preferably, the anhydrous composition further comprises at least one dimethicone derivative, selected from lauryl dimethicone, cetyl dimethicone, cetearyl dimethicone, behenyl dimethicone, C24-28 alkyl dimethicone, bis-hydroxyethoxypropyl dimethicone, phenyl propyl silsesquisiloxane, dimethiconol stearate, hydroxypropyl dimethicone behenate, and mixtures thereof. More preferably, the anhydrous composition further comprises cetyl dimethicone.

Preferably, the anhydrous composition further comprises 10-40 wt% of said at least one dimethicone derivative, based on the weight of the anhydrous composition, and more preferably 20-40 wt%.

Preferably, the anhydrous composition further comprises caprylyl methicone.

Preferably, the anhydrous composition further comprises 10-30 wt% of caprylyl methicone, based on the weight of the anhydrous composition, and more preferably 10-30 wt%.

Preferably, the anhydrous composition further comprises trimethylsiloxysilicate.

Preferably, the anhydrous composition further comprises 1-5 wt% of trimethylsiloxysilicate, based on the weight of the anhydrous composition, and more preferably 1-3 wt%.

The anhydrous composition may also include at least one pigment. The choice of the pigment and the ability thereof to be adequately dispersed and appropriately suspended are essential to produce a good quality product. In addition to the usual colouring pigments, other colour tones may be added depending on the desired product colour, tone, and reflection. Products based on natural and synthetic mica, borosilicates, or bismuth oxychloride (known as "pearl essence"), are common white, iridescent, or coloured additives, which can also be mixed with gold, silver, and bronze tones.

Preferably, said at least one pigment is coated with a hydrophobic material, preferably a silicone hydrophobic material. Suitable silicon hydrophobic materials include triethoxy-caprylyl silane, dimethicone, crosslinked polymer of dimethoxy-diphenyl-silane-triethoxy-caprylyl silane, octyltriethoxysilane, and mixtures thereof.

The spheres of anhydrous composition suspended in the aqueous gel can have essentially the same diameter or different diameters. The choice of diameter depends on the aesthetic effect desired for the end cosmetic product.

The spheres of anhydrous composition have a diameter of 1 to 8 mm.

In some embodiments, the spheres have a diameter of about 3-6 mm.

In other embodiments, the spheres have a diameter of about 1-3 mm.

With respect to the aqueous gel, said polyacrylate may be polyacrylic acid (also known as Carbomer), potassium aluminium polyacrylate, potassium polyacrylate, sodium polyacrylate, ammonium polyacrylate, glyceryl polyacrylate, or a mixture thereof.

Preferably, said polyacrylate is glyceryl polyacrylate.

Preferably, the aqueous gel comprises 10-35 wt% of said polyacrylate, based on the weight of the aqueous gel, more preferably 10-25 wt%. Said percentage allows to obtain the desired Brookfield viscosity of 0.2-0.9 Pa*s, at 20 rpm and 25 °C.

Preferably, the aqueous gel comprises at least 50 wt% of water, based on the weight of the aqueous gel, more preferably 60-75 wt%.

Preferably, said aqueous gel further comprises at least one water-miscible solvent, and more preferably an alcoholic solvent, such as ethanol.

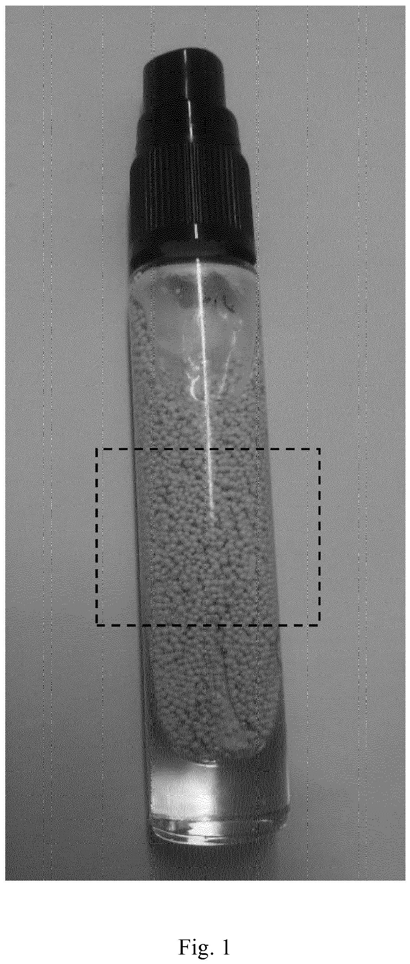

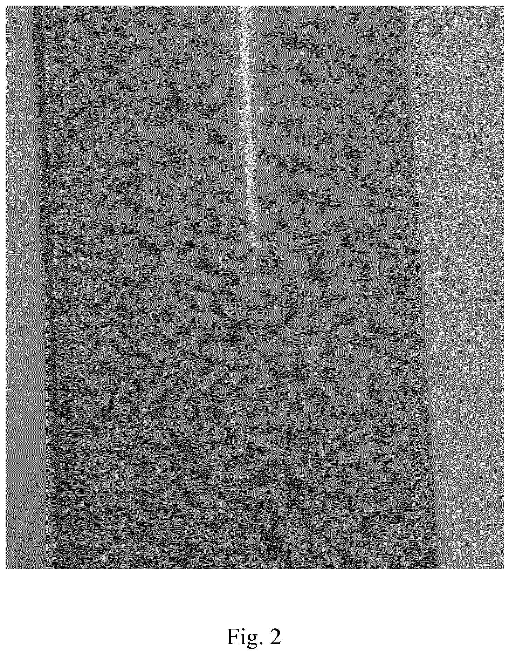

The cosmetic product according to the invention therefore originates from the combination of two wholly incompatible phases, i.e. an aqueous gel and a preferably coloured anhydrous composition. Once combined, the anhydrous composition forms spheres which remain suspended in the transparent aqueous gel, thus creating an aesthetic effect, such as that shown in Figure 1 and Figure 2, wherein the anhydrous composition forms spheres with different diameters. During application of the product, the two phases are taken out of the container simultaneously, and - once applied to the skin - create a thin, uniform film. They may be taken out with a spatula, a paddle brush, a wand, an eye shadow applicator, a round brush, a sponge, or a pump delivery system.

Accordingly, the cosmetic product envisaged in the invention is particularly suitable for the preparation of articles for makeup, such as eye shadows, foundations, bronzers, blushers, correctors, mascara, highlighting face powders, lip glosses, and face primers.

The present invention therefore also relates to an article for make-up comprising a transparent container, preferably made of glass, wherein the above described cosmetic product is contained.

Indeed, the transparency of the container is advisable in order to highlight the presence of the spheres in said cosmetic product. Glass is the most suitable material for this purpose.

In another aspect, the present invention relates to a process for preparing the cosmetic product as described above. This process comprises the following steps:
a) providing an aqueous gel,
b) providing an anhydrous composition,
c) adding the anhydrous composition to the aqueous gel, said gel being under mechanical or magnetic stirring, thereby obtaining the formation of spheres of anhydrous composition in the aqueous gel.

Preferably, the resulting cosmetic product is kept under stirring for a few minutes after the addition of the anhydrous composition.

The power applied during the stirring phase allows to control the size of the spheres. Lower power results in the formation of larger spheres, for example, of 3-6 mm. Higher power results in the formation of smaller spheres, for example, of 1-3 mm.

The duration and speed of the stirring therefore allow the final size of the spheres to be controlled.

Spheres with a larger diameter are obtained with a stirring speed of 100-400 rpm, applied for a time interval of 30 sec to 2 min.

Spheres with a smaller diameter are obtained with a stirring speed of 300-600 rpm, applied for a time interval of 30 sec to 2 min.

The cosmetic product thus obtained is stable over time and shows the clearly defined spheres formed within the aqueous gel, which are also clearly visible in Figures 1 and 2.

It should also be understood that all aspects identified as preferred and advantageous for the cosmetic product should be deemed equally preferable and advantageous also for the uses and the preparation process thereof.

It should likewise be understood that all combinations of the preferred aspects of the cosmetic product envisaged in the invention, as well as the uses and the preparation process thereof, as set out above, should be considered as described herein.

Various embodiments of the innovation have been disclosed herein, but further embodiments may also be conceived using the same innovative concept.

Below are working examples of the present invention provided for illustrative purposes.

### EXAMPLES

### Example 1.

A highlighting face powder was prepared wherein the aqueous gel had the following composition:

| ingredient | wt% |
|---|---|
| water | 72.7875 |
| ethylhexylglycerin | 0.0375 |
| glyceryl polyacrylate | 12.25 |
| phenoxyethanol | 0.825 |
| ethanol | 5 |
| butylene glycol | 5 |
| pentylene glycol | 4 |
| disodium EDTA | 0.1 |
| total | 100 |

and the anhydrous composition had the following composition:

| ingredient | wt% |
|---|---|
| Cetyl dimethicone | 33.9474 |
| Dimethicone | 21.25 |
| *Dimethicone crosspolymer* | 3.75 |
| Caprylyl methicone | 19.7368 |
| Trimethylsiloxysilicate | 1.3158 |
| Triethoxycaprylylsilane-coated beads | 20 |
| Total | 100 |

The anhydrous composition was added to the aqueous gel, which had been preliminarily placed under mechanical stirring, thus obtaining the formation of spheres of anhydrous composition in aqueous gel.

The resulting cosmetic product was kept under controlled stirring for the duration necessary to obtain the desired size of the beads once the anhydrous composition had been added completely.

The resulting cosmetic product is shown in Figures 1 and 2. It was observed that the spheres of anhydrous composition had a well-defined surface and were stable in the aqueous gel.

## Claims

1. A cosmetic product comprising spheres of an anhydrous composition suspended in an aqueous gel, wherein:
said anhydrous composition comprises 10-65 wt% of a mixture of dimethicone and dimethicone crosspolymer, based on the weight of the anhydrous composition, and optionally at least one pigment,
said aqueous gel comprises water and polyacrylate, said aqueous gel having a Brookfield viscosity of 0.2-0.9 Pa*s, at 20 rpm and 25°C, and
said spheres of anhydrous composition have a diameter of about 1 mm up to 8 mm.

2. The cosmetic product of claim 1, wherein the spheres of an anhydrous composition are present in the cosmetic product in an amount less than or equal to the aqueous gel, preferably the spheres of an anhydrous composition are present in the cosmetic product in an amount not higher than 50 wt%, based on the weight of the cosmetic product.

3. The cosmetic product of claim 1 or 2, wherein, in said mixture, dimethicone and dimethicone crosspolymer are in a weight ratio of 20:1 to 2:1, preferably 10:1 to 2:1, more preferably 8:1 to 3:1.

4. The cosmetic product of any one of claims 1-3, wherein the mixture of dimethicone and dimethicone crosspolymer has a kinematic viscosity of 300-600 m²/s, at 25°C.

5. The cosmetic product of any one of claims 1-4, wherein said anhydrous composition comprises 10-60 wt% of a mixture of dimethicone and dimethicone crosspolymer, based on the weight of the anhydrous composition.

6. The cosmetic product of any one of claims 1-5, wherein the anhydrous composition further comprises at least one dimethicone derivative selected from lauryl dimethicone, cetyl dimethicone, cetearyl dimethicone, behenyl dimethicone, C24-28 alkyl dimethicone, bishydroxyethoxy propyl dimethicone, phenyl propyl silsesquisiloxane, dimethiconol stearate, hydroxy propyl dimethicone behenate, and mixtures thereof.

7. The cosmetic product of any one of claims 1-6, wherein the anhydrous composition further comprises caprilyl methicon.

8. The cosmetic product of any one of claims 1-7, wherein, in the aqueous gel, said polyacrylate is polyacrylic acid, potassium aluminum polyacrylate, potassium polyacrylate, sodium polyacrylate, ammonium polyacrylate, glyceryl polyacrylate, or a mixture thereof, preferably it is glyceryl polyacrylate.

9. The cosmetic product of any one of claims 1-8, wherein the aqueous gel comprises 10-35 wt% of said polyacrylate, based on the weight of the aqueous gel, preferably 10-25 wt%.

10. A make-up article comprising a transparent container, preferably of glass, wherein the cosmetic product of any one of claims 1-9 is present.

## Patentansprüche

1. Kosmetisches Produkt, umfassend Kugeln einer wasserfreien Zusammensetzung, die in einem wässrigen Gel suspendiert sind, wobei:
die wasserfreie Zusammensetzung 10-65 Gew.-% einer Mischung aus Dimethicon und Dimethicon-Kreuzpolymer, bezogen auf das Gewicht der wasserfreien Zusammensetzung, und optional wenigstens ein Pigment umfasst,
das wässrige Gel Wasser und Polyacrylat umfasst, wobei das wässrige Gel eine Brookfield-Viskosität von 0,2-0,9 Pa*s bei 20 U/min und 25°C aufweist, und
die Kugeln aus wasserfreier Zusammensetzung einen Durchmesser von etwa 1 mm bis 8 mm aufweisen.

2. Kosmetisches Produkt nach Anspruch 1, wobei die Kugeln einer wasserfreien Zusammensetzung in dem kosmetischen Produkt in einer Menge kleiner oder gleich dem wässrigen Gel vorhanden sind, wobei vorzugsweise die Kugeln einer wasserfreien Zusammensetzung in dem kosmetischen Produkt in einer Menge nicht höher als 50 Gew.-% vorhanden sind, bezogen auf das Gewicht des kosmetischen Produkts.

3. Kosmetisches Produkt nach Anspruch 1 oder 2, wobei in der Mischung Dimethicon und Dimethicon-Kreuzpolymer in einem Gewichtsverhältnis von 20 : 1 bis 2 : 1, vorzugsweise 10 : 1 bis 2 : 1, stärker bevorzugt 8 : 1 bis 3 : 1 vorliegen.

4. Kosmetisches Produkt nach einem der Ansprüche 1-3, wobei die Mischung aus Dimethicon und Dimethicon-Kreuzpolymer eine kinematische Viskosität von 300-600 m²/s bei 25 °C aufweist.

5. Kosmetisches Produkt nach einem der Ansprüche 1-4, wobei die wasserfreie Zusammensetzung 10-60 Gew.-% einer Mischung aus Dimethicon und Dimethicon-Kreuzpolymer umfasst, bezogen auf das Gewicht der wasserfreien Zusammensetzung.

6. Kosmetisches Produkt nach einem der Ansprüche 1-5, wobei die wasserfreie Zusammensetzung ferner wenigstens ein Dimethicon-Derivat umfasst, ausgewählt aus Lauryldimethicon, Cetyldimethicon, Cetearyldimethicon, Behenyldimethicon, C24-28-Alkyldimethicon, Bishydroxyethoxypropyldimethicon, Phenylpropylsilsesquisiloxan, Dimethiconolstearat, Hydroxypropyldimethiconbehenat und Mischungen davon.

7. Kosmetisches Produkt nach einem der Ansprüche 1-6, wobei die wasserfreie Zusammensetzung ferner Caprilylmethicon umfasst.

8. Kosmetisches Produkt nach einem der Ansprüche 1-7, wobei das Polyacrylat in dem wässrigen Gel Polyacrylsäure, Kaliumaluminiumpolyacrylat, Kaliumpolyacrylat, Natriumpolyacrylat, Ammoniumpolyacrylat, Glycerylpolyacrylat oder eine Mischung davon ist, vorzugsweise Glycerylpolyacrylat.

9. Kosmetisches Produkt nach einem der Ansprüche 1 bis 8, wobei das wässrige Gel 10-35 Gew.-% des Polyacrylats, bezogen auf das Gewicht des wässrigen Gels, vorzugsweise 10-25 Gew.-% umfasst.

10. Schminkerzeugnis, umfassend einen transparenten Behälter, vorzugsweise aus Glas, wobei das kosmetische Produkt nach einem der Ansprüche 1-9 vorhanden ist.

## Revendications

1. Produit cosmétique comprenant des billes d'une composition anhydre en suspension dans un gel aqueux :
ladite composition anhydre comprenant 10 à 65 % en poids d'un mélange de diméthicone et de polymère réticulé de diméthicone, par rapport au poids de la composition anhydre, et éventuellement au moins un pigment,
ledit gel aqueux comprenant de l'eau et du polyacrylate, ledit gel aqueux ayant une viscosité Brookfield de 0,2 à 0,9 Pa*s, à 20 tr/min et 25°C, et
lesdites billes de composition anhydre ayant un diamètre d'environ 1 mm jusqu'à 8 mm.

2. Produit cosmétique selon la revendication 1, lesdites billes d'une composition anhydre étant présentes dans le produit cosmétique en une quantité inférieure ou égale à celle du gel aqueux, de préférence les billes d'une composition anhydre étant présentes dans le produit cosmétique en une quantité non supérieure à 50 % en poids, par rapport au poids du produit cosmétique.

3. Produit cosmétique selon la revendication 1 ou 2, dans ledit mélange, ladite diméthicone et ledit polymère réticulé de diméthicone étant dans un rapport massique de 20:1 à 2:1, de préférence de 10:1 à 2:1, plus préférablement de 8:1 à 3:1.

4. Produit cosmétique selon l'une quelconque des revendications 1 à 3, ledit mélange de diméthicone et de polymère réticulé de diméthicone ayant une viscosité cinématique de 300 à 600 m²/s, à 25°C.

5. Produit cosmétique selon l'une quelconque des revendications 1 à 4, ladite composition anhydre comprenant 10 à 60 % en poids d'un mélange de diméthicone et de polymère réticulé de diméthicone, par rapport au poids de la composition anhydre.

6. Produit cosmétique selon l'une quelconque des revendications 1 à 5, ladite composition anhydre comprenant en outre au moins un dérivé de diméthicone choisi parmi la lauryldiméthicone, la cétyldiméthicone, la cétéaryldiméthicone, la béhényldiméthicone, une (C24 à C28)alkyldiméthicone, la bishydroxyéthoxypropyldiméthicone, le phénylpropylsilsesquisiloxane, le stéarate de diméthiconol, le béhénate d'hydroxypropyldiméthicone et les mélanges de ceux-ci.

7. Produit cosmétique selon l'une quelconque des revendications 1 à 6, ladite composition anhydre comprenant en outre de la caprilylméthicone.

8. Produit cosmétique selon l'une quelconque des revendications 1 à 7, dans le gel aqueux, ledit polyacrylate étant l'acide polyacrylique, le polyacrylate d'aluminium et de potassium, le polyacrylate de potassium, le polyacrylate de sodium, le polyacrylate d'ammonium, le polyacrylate de glycéryle, ou un mélange de ceux-ci, de préférence celui-ci étant le polyacrylate de glycéryle.

9. Produit cosmétique selon l'une quelconque des revendications 1 à 8, ledit gel aqueux comprenant 10 à 35 % en poids dudit polyacrylate, par rapport au poids du gel aqueux, de préférence 10 à 25 % en poids.

10. Article de maquillage comprenant un récipient transparent, de préférence en verre, ledit produit cosmétique selon l'une quelconque des revendications 1 à 9 étant présent.
